# EUROPEAN PATENT APPLICATION

(11) **EP 1 913 969 A1**
(43) Date of publication of application: **23.04.2008**
(21) Application number: 06782468.0
(22) Date of filing: 08.08.2006
(51) Int. Cl.: A61N 1/30, A61N 1/04

(54) **IONTOPHORESIS APPARATUS**

(30) Priority: 08.08.2005 JP 2005229985
(71) Applicant: Transcu Ltd., 048580 Singapore (SG)
(72) Inventor: KANAMURA, Kiyoshi, Tokyo 150-0022 (JP); MATSUMURA, Takehiko, Tokyo 150-0022 (JP); NAKAYAMA, Mizuo, Tokyo 150-0022 (JP); AKIYAMA, Hidero, Tokyo 150-0022 (JP); MATSUMURA, Akihiko, Tokyo 150-0022 (JP)
(74) Representative: Schäfer, Matthias W.
(86) International application number: PCT/JP2006/315627
(87) International publication number: WO 2007/018197

(57) **Abstract**

Provided is an iontophoresis device. In the device, a doping layer is provided on an electrode in a working electrode structure or non-working electrode structure of the device. The doping layer comprises a substance which can cause an electrochemical reaction by doping or dedoping of an ion (e.g., an ion of a conductive polymer). The device can prevent or at least reduce the generation of a gas or undesirable ion which may be caused by the electrode reaction in an electrode structure or the denaturation of a medicinal substance which may be caused by any chemical reaction during energization of the device.

## Description

### FIELD OF THE INVENTION

The present invention relates to an iontophoresis device. In particular, the present invention relates to an iontophoresis device capable of preventing or suppressing an unpreferable electrode reaction in an electrode assembly.

### BACKGROUND OF THE INVENTION

Iontophoresis involves electrically driving a drug dissociated to plus or minus ions in a solution by means of a voltage to transdermally transfer the drug into an organism, and has advantages such as a reduced load to a patient and excellent controllability of the amount of the drug to be administered.

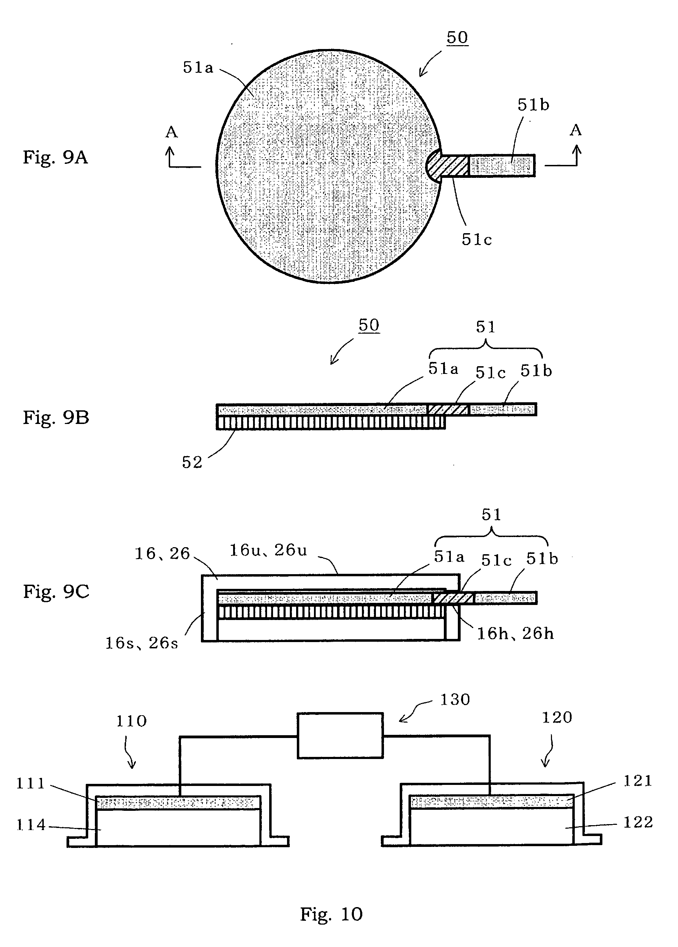

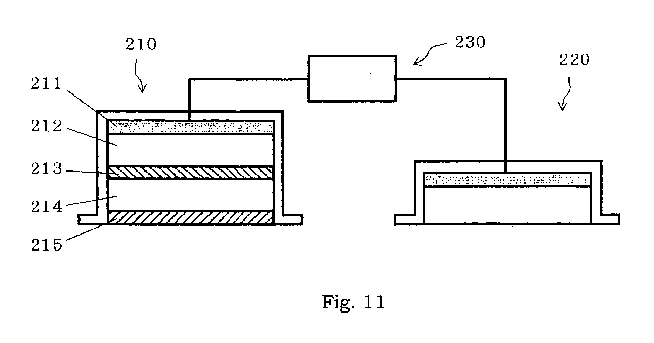

Fig. 9 is an explanatory view showing the basic constitution of an iontophoresis device as a device for performing the above-mentioned iontophoresis.

As shown in the figure, the iontophoresis device includes: a working electrode assembly 110 having an electrode 111 and a drug solution holding portion 114 holding a solution of a drug that dissociates to plus or minus drug ions (a drug solution) ; a non-working electrode assembly 120 having an electrode 121 and an electrolyte solution holding portion 122 holding an electrolyte solution; and an electric power source 130 with both of its terminals connected to the electrodes 111 and 121. A voltage having the same conductivity type as that of a drug ion is applied to the electrode 111 and a voltage having a conductivity type opposite to that of the drug ion is applied to the electrode 121 in a state where the drug solution holding portion 114 and the electrolyte solution holding portion 122 are brought into contact with the skin of an organism, whereby the drug ion is administered to the organism.

One of the problems to be solved in such iontophoresis device is various electrode reactions occurring in the electrode assemblies 110 and 120.

For example, in the case where a drug is a cationic drug that dissociates to plus drug ions, a hydrogen ion or an oxygen gas may be generated at the electrode 111 and a hydroxide ion or a hydrogen gas may be generated at the electrode 121 by the electrolysis of water. In addition, the drug causes a chemical reaction near the electrode 111 to alter upon energization depending on the kind of the drug and energization conditions. Furthermore, when the drug solutionholding portion 114 contains a chlorine ion, a chlorine gas or hypochlorous acid may be generated.

Similarly, in the case where a drug is an anionic drug that dissociates to minus drug ions, a hydroxide ion or a hydrogen gas may be generated at the electrode 111 and a hydrogen ion or an oxygen gas may be generated at the electrode 121 by the electrolysis of water. In addition, the drug causes a chemical reaction near the electrode 111 to alter upon energization depending on the kind of the drug and energization conditions. Furthermore, when the electrolyte solution holding portion 122 contains a chlorine ion, a chlorine gas or hypochlorous acid may be generated.

When such gas as described above is generated in the electrode assembly 110 or 120, energization from the electrode 111 or 121 to the drug solution or the electrolyte solution is inhibited. When a hydrogen ion, a hydroxide ion, and hypochlorous acid are generated in the electrode assembly 110 or 120, they transfer to a biological interface to have a detrimental effect on an organism. In addition, the alteration of a drug may cause unpreferable conditions such as the inability to obtain an initial drug effect and the production of a toxic substance.

Patent Document 1 discloses, as an iontophoresis device capable of solving such problems as described above, an iontophoresis device in which a silver electrode is used as an anode and a silver chloride electrode is used as a cathode.

In the iontophoresis device, a reaction preferentially occurs, in which silver in the anode is oxidized by energization to become insoluble silver chloride, while silver chloride is reduced at the cathode to become metal silver. As a result, the generation of various gases and the production of various ions due to such electrode reactions as described above can be suppressed.

However, it is difficult to prevent the dissolution of the silver electrode during storage of the iontophoresis device. In particular, in the case where the device is intended for administering a cationic drug, the number of kinds of applicable drugs is extremely limited. In addition, a morphological change upon production of silver chloride from the silver electrode is large. Therefore, special consideration must be given in order to prevent such morphological change from affecting the properties of the device. As a result, there arises a problem in that a severe restriction is imposed on the shape of the device (for example, a lamination structure cannot be adopted). Furthermore, the iontophoresis device is unable to solve the problem of the alteration of a drug upon energization.

Patent Document 2 discloses, as another iontophoresis device capable of solving the above problems, an iontophoresis device shown in Fig. 10.

As shown in the figure, the iontophoresis device is constituted by: a working electrode assembly 210 including an electrode 211, an electrolyte solution holding portion 212 holding an electrolyte solution in contact the electrode 211, an ion exchange membrane 213 of a second conductivity type, the ion exchange membrane 213 being placed on the front surface side of the electrolyte solution holding portion 212, a drug solution holding portion 214 holding a drug solution containing a drug ion of a first conductivity type, the drug solution holding portion 214 being placed on the front surface side of the ion exchange membrane 213, and an ion exchange membrane 215 of the first conductivity type, the ion exchange membrane 215 being placed on the front surface side of the drug solution holding portion 214; and a non-working electrode assembly 220 and an electric power source 230 similar to those shown in Fig. 9.

In the iontophoresis device, the electrolyte solution and the drug solution are partitioned by the second ion exchange membrane 213 of the second conductivity type. As a result, the composition of the electrolyte solution can be selected independently of the drug solution. Accordingly, an electrolyte solution containing no chlorine ion can be used, and the selection of an electrolyte having a lower oxidation or reduction potential than the electrolysis of water as the electrolyte in the electrolyte solution can suppress the production of an oxygen gas, a hydrogen gas, a hydrogen ion, or a hydroxide ion resulting from the electrolysis of water. Alternatively, the use of a buffer electrolyte solution into which multiple kinds of electrolytes are dissolved can suppress a change in pH due to the production of a hydrogen ion or a hydroxide ion. Furthermore, in the iontophoresis device, the transfer of a drug ion to the electrolyte solution holding portion is blocked by the second ion exchange membrane, so a problem in that the drug alters owing to a chemical reaction upon energization is solved.

On the other hand, the iontophoresis device disclosed in Patent Document 2 is constituted by a large number of members, and each of the electrolyte solution holding portion 212 and the drug solution holding portion 214 must be handled in a wet state (a state with a high water content). Therefore, there arises a problem in that the automation of the production of the device and the mass production of the device are difficult or that a production cost is hardly reduced.

Patent Document 1: US 4,744,787
Patent Document 2: JP 3040517 B
Non-Patent Document 1: "KS Kagaku Senmonsho Dodensei Kobunshi" edited by Naoya Ogata, Kodansha, published on January, 1990
Non-Patent Document 2: "Shin Zairyou series Dodensei Koubunshi no Saishin Ouyou Gijutsu" written by Yukuo Kobayashi, CMC Publishing CO., LTD., published on July, 2004

### DISCLOSURE OF INVENTION

### PROBLEMS TO BE SOLVED

An object of the present invention is to provide an iontophoresis device capable of preventing or suppressing the generation of an oxygen gas, a chlorine gas, or a hydrogen gas in an electrode assembly.

Another object of the present invention is to provide an iontophoresis device capable of preventing or suppressing the generation of a hydrogen ion, a hydroxide ion, or hypochlorous acid in an electrode assembly.

Another object of the present invention is to provide an iontophoresis device capable of preventing or suppressing the alteration of a drug due to a chemical reaction upon energization.

Another object of the present invention is to provide an iontophoresis device which is capable of preventing or suppressing the generation of such gas or ion as described above or the alteration of a drug and which does not cause a large morphological change to an electrode due to energization.

Another object of the present invention is to provide an iontophoresis device which is capable of preventing or suppressing the generation of such gas or ion as described above or the alteration of a drug and which has a simplified structure.

Another object of the present invention is to provide an iontophoresis device which is capable of preventing or suppressing the generation of such gas or ion as described above or the alteration of a drug and the automation of the production of which or the mass production of which is easily performed.

Another object of the present invention is to provide an iontophoresis device which is capable of preventing or suppressing the generation of such gas or ion as described above or the alteration of a drug and the production cost of which can be reduced.

### MEANS FOR SOLVING PROBLEMS

According to one aspect of the present invention, there is provided an iontophoresis device characterized by including at least one electrode assembly having an electrode in which a doping layer made of a substance effecting an electrochemical reaction owing to the doping or de-doping of an ion is formed.

In the present invention, the electrode possessed by the electrode assembly has a doping layer as a layer of a substance effecting an electrochemical reaction owing to the doping or de-doping of an ion (hereinafter, an electrode having such doping layer may be referred to as the "doping electrode").

Therefore, energization from an electric power source to an electrolyte solution or a drug solution is entirely or mostly caused by the doping of a doping layer with an ion or the de-doping of the ion from the layer. As a result, an electrode reaction generating a gas such as an oxygen gas, a chlorine gas, or a hydrogen gas, or an unpreferable ion such as a hydrogen ion, a hydroxide ion, or hypochlorous acid can be prevented or at least reduced.

The term "effecting an electrochemical reaction owing to the doping of an ion" refers to the fact that, when plus or minus charge is given to a doping layer, the doping layer captures an ion charged to the polarity opposite to the charge from an electrolyte solution or drug solution in contact with the doping layer, and the layer is doped with the ion (the ion binds to a substance constituting the doping layer), so the given charge is compensated. The term "effecting an electrochemical reaction owing to the de-doping of an ion" refers to the fact that, when plus charge is given to the doping layer, a plus ion with which the doping layer is doped is de-doped and released from the doping layer, so the given charge is compensated, or the fact that, when minus charge is given to the doping layer, a minus ion with which the doping layer is doped is de-doped and released from the doping layer, so the given charge is compensated.

Polyaniline, polypyrrole, polythiophene, or polyacetylene as a conductive polymer, or a derivative of each of them, or a mixture of them is typically used as a material for the doping layer in the present invention. Of those, polyaniline is most preferably used. Non-Patent Documents 1 and 2 detail the derivatives of polyaniline, polypyrrole, polythiophene, or polyacetylene that can be used for the doping layer of the present invention.

Various methods have been known as a method of producing a conductive polymer or a method of forming a conductive polymer into a film. Examples of the methods include: a method involving subjecting a powder-like conductive polymer chemically synthesized by means of an oxidation polymerization method to compression molding; a method involving bringing a conductive polymer into an ink state by means of a polar organic solvent such as N-methylpyrrolidone, molding the ink-like polymer, and removing the solvent; and a method involving immersing an appropriate conductive base material in a solution of a monomer for producing a conductive polymer and performing electrolytic polymerization to form a conductive polymer layer on the base material. The doping layer of the present invention can be formed by means of any one of those arbitrary methods.

In this case, the entirety of the doping layer may be constituted only by the conductive polymer, or the doping layer may contain a component except the conductive polymer. For example, in order to impart mechanical strength against tear or break to the doping layer, an appropriate woven or non-woven fabric impregnated with the conductive polymer can be used, or the conductive polymer and an appropriate polymer binder can be blended with each other. Alternatively, in order to improve the conductivity of the conductive polymer, the conductive polymer can be blended with a conductive filler such as carbon.

As described below, the conductive polymer may be doped with a drug ion to be administered to an organism or with an ion for substituting a drug ion with which the first ion exchange membrane is doped. In addition, the conductive polymer can be doped with an ion as an electron acceptor or an electron donor for the purpose of improving the conductivity of the conductive polymer.

Examples of a material that can be used for the doping layer of the present invention except the conductive polymer include carbon materials such as black lead and graphite.

As described below with respect to the invention according to claim 14, the electrode assembly having the doping electrode can be directly used as a non-working electrode assembly. Simultaneously, the electrode assembly having the doping electrode can be used also as a working electrode assembly by doping the doping layer with a drug ion before use.

That is, energization is performed by applying a voltage having the conductivity type opposite to that of a drug ion to the doping electrode in a state where the doping layer is immersed in a drug solution containing the drug ion at an appropriate, whereby the doping layer can be doped with the drug ion.

In addition, the drug ion can be administered to an organism by applying a voltage having the same conductivity type as that of the drug ion to the doping electrode in a state where the doping layer doped with the drug ion is brought into contact with the skin of the organism.

In this case, energization from the doping electrode to the organism is entirely or partially caused by the de-doping of the drug ion from the doping layer to transfer to the organism. As a result, the production of the above-described gas or unpreferable ion is prevented or at least reduced.

Furthermore, the doping layer doped with the drug ion functions as an ion exchange membrane of the same conductivity type as that of the drug ion. That is, doping the doping layer with a plus drug ion imparts, to the doping layer, an ion exchange function of permitting the passage of a plus ion and blocking the passage of a minus ion. Similarly, doping the doping layer with a minus drug ion imparts, to the doping layer, an ion exchange function of permitting the passage of a minus ion and blocking the passage of a plus ion.

Therefore, upon administration of the drug ion to an organism, the transfer of an organism counter ion (an ion present on the surface of the organism or in the organism, the ion being charged to the conductivity type opposite to that of the drug ion) to the doping layer is blocked, whereby the amount of a current consumed can be reduced, and the efficiency of administration of a drug can be increased.

The doping of the doping layer with the drug ion in the case where the electrode assembly having the doping electrode is used as a working electrode assembly in the manner as that described above may be performed at any time during the period commencing on the stage of the production of the iontophoresis device or the working electrode assembly and ending on the stage immediately before use (the administration of a drug to an organism).

As described above, an iontophoresis device typically includes a working electrode assembly holding a drug to be administered to an organism and a non-working electrode assembly serving as a counter electrode of the working electrode assembly. In this case, in the iontophoresis device of the present invention, at least one of the working electrode assembly and the non-working electrode assembly is an electrode assembly having a doping electrode, or each of the electrode assemblies is preferably an electrode assembly having a doping electrode.

Depending on the kind of an iontophoresis device, a drug to be administered to an organism may be held by each of two electrode assemblies to be connected to both polarities of an electric power source (in this case, each of both electrode assemblies serves as a working electrode assembly and a non-working electrode assembly), or multiple electrode assemblies may be connected to each polarity of an electric power source. In such case, in the iontophoresis device of the present invention, at least one of the electrode assemblies is an electrode assembly having a doping electrode, or all the electrode assemblies are preferably electrode assemblies each having a doping electrode.

An electrode assembly having a doping electrode has an extremely simple structure. Therefore, the automation of the production of an iontophoresis device including an electrode assembly having a doping electrode as at least one of a working electrode assembly and a non-working electrode assembly and the mass production of the device can be easily performed, and the production cost of the device can be significantly reduced.

In the invention according to any one of claims 1 to 3, the electrode assembly can further include a drug solution holding portion holding a drug solution containing a drug ion of the first conductivity type, the drug solution holding portion being placed on the front surface side of the doping layer (claim 4).

Such electrode assembly can be used as a working electrode assembly in an iontophoresis device. A drug ion in the drug solution holding portion is administered to an organism by applying a voltage of the first conductivity type to the doping electrode in a state where the drug solution holding portion is brought into contact with the skin of the organism.

In this case, the doping layer captures an ion of a second conductivity type from the drug solution holding portion, and the layer is doped with the ion, whereby energization from the doping electrode to the drug solution holding portion occurs. Therefore, the generation of the above-described gas or unpreferable ion can be suppressed.

The doping layer can be doped with an ion of the first conductivity type in advance. In this case, energization from the doping electrode to the drug solution holding portion is caused by the doping with an ion of the second conductivity type in the drug solution holding portion and the de-doping of an ion of the first conductivity type from the doping layer. It should be noted that the same holds true for the invention according to claim 7 or the like in this respect.

The doping layer can be doped with an ion of the first conductivity type through energization as a result of applying a voltage of the second conductivity type to the doping electrode in a state where the doping layer is immersed in an electrolyte solution containing an appropriate concentration of the ion of the first conductivity type.

In the invention according to claim 4, the electrode assembly preferably further includes a first ion exchange membrane of the first conductivity type placed on the front surface side of the drug solution holding portion (claim 5).

In the electrode assembly thus constituted, the drug ion in the drug solution holding portion is administered to an organism through the first ion exchange membrane by applying a voltage of the first conductivity type to the doping electrode in a state where the first ion exchange membrane is brought into contact with the skin of the organism.

In this case, an additional effect, that is, an increase in efficiency of administration of the drug ion can be obtained because the transfer of an organism counter ion to the drug solution holding portion is blocked by the first ion exchange membrane.

In the invention according to claim 4 or 5, it is preferable that: the electrode assembly further include a second ion exchange membrane of the second conductivity type placed on the front surface side of the doping layer; and the drug solution holding portion be placed on the front surface side of the second ion exchange membrane (claim 6).
In the electrode assembly thus constituted, a drug is administered to an organism in the same manner as that described above. In addition, the alteration of the drug near the doping electrode during energization is prevented or suppressed because the second ion exchange membrane blocks the transfer of a drug ion to the side of the doping electrode.

In this case, the second ion exchange membrane and the doping layer are preferably joined integrally with each other. The integral joining can improve energization property between the doping layer and the second ion exchange membrane and simplify the assembly work of the electrode assembly. Therefore, the automation of the production of the electrode assembly and the mass production of the electrode assembly can be easily performed, or a reduction in production cost can be achieved.

The second ion exchange membrane and the doping layer can be joined with each other by, for example, thermocompression bonding. Alternatively, the joining can be performed by forming the doping layer by means of any one of the above-described various methods on the second ion exchange membrane.

In the invention according to any one of claims 1 to 3, the electrode assembly can further include: an electrolyte solution holding portion holding an electrolyte solution, the electrolyte solution holding portion being placed on the front surface side of the doping layer; and a first ion exchange membrane of the first conductivity type that is placed on the front surface side of the electrolyte solution holding portion and that is doped with a drug ion of the first conductivity type (claim 7).

Such electrode assembly can be used as a working electrode assembly in an iontophoresis device. A drug ion with which the first ion exchange membrane is doped can be administered to an organism by applying a voltage of the first conductivity type to the doping electrode in a state where the first ion exchange membrane is brought into contact with the skin of the organism.

Here, the electrolyte solution of the electrolyte solution holding portion serves to supply an ion of the first conductivity type for substituting the drug ion in the first ion exchange membrane (hereinafter, the ion of the first conductivity type in the electrolyte solution is referred to as the "first electrolytic ion") and to supply an ion of the second conductivity type with which the doping layer is to be doped (hereinafter, the ion of the second conductivity type in the electrolyte solution is referred to as the "second electrolytic ion").

That is, the doping layer captures the second electrolytic ion, and the layer is doped with the ion, whereby energization from the doping electrode to the electrolyte solution holding portion occurs. In addition, the drug ion in the first ion exchange membrane is substituted by the first electrolytic ion from the electrolyte solution holding portion, so it can transfer to an organism.

In the present invention, the first ion exchange membrane can be doped with a drug ion by immersing the first ion exchange membrane in a drug solution containing an appropriate concentration of drug ion for a predetermined time period. The amount of the drug ion with which the first ion exchange membrane is to be doped can be controlled by adjusting the concentration of the drug ion, an immersion time, and the number of times of immersion in this case.

When the first electrolytic ion has a mobility larger than that of a drug ion, a higher priority may be placed on the transfer of the first electrolytic ion to an organism than that on the transfer of the drug ion to the organism, so the efficiency of administration of a drug may reduce. Therefore, composition in which the first electrolytic ion has a mobility comparable to or smaller than that of the drug ion is preferably selected for the electrolyte solution of the electrolyte solution holding portion. Alternatively, such reduction in efficiency of administration as described above can be prevented by using the drug ion as the first electrolytic ion of the electrolyte solution holding portion.

In the present invention, the efficiency of administration of a drug ion can be increased because the first ion exchange membrane blocks the transfer of an organism counter ion to the electrolyte solution holding portion. Furthermore, the efficiency of administration of the drug ion can be additionally increased because the drug ion is held by the first ion exchange membrane as a member to be brought into direct contact with the skin of an organism.

In addition, the stability of the drug ion during storage is improved, and the amount of a stabilizer, an antimicrobial agent, an antiseptic, or the like to be used can be reduced or the storage period of the device can be prolonged because the first ion exchange membrane holds the drug ion by being doped with the ion (that is, the drug ion binds to an ion exchange group in the ion exchange membrane) . In addition, the stability of the administration of a drug can be improved because the amount of the drug ion with which the first ion exchange membrane is to be doped can be strictly adjusted. Furthermore, the assembly work of the electrode assembly can be simplified because the first ion exchange membrane with which the drug ion is doped is used instead of the drug solution holding portion that must have been conventionally used in a wet state. Therefore, the automation of the production of the electrode assembly and the mass production of the electrode assembly can be easily performed, or a reduction in production cost can be achieved.

In the invention according to claim 7, it is preferable that: the electrode assembly further include a second ion exchange membrane of the second conductivity type placed on the front surface side of the electrolyte solution holding portion; and the first ion exchange membrane be placed on the front surface side of the second ion exchange membrane (claim 8).

In such electrode assembly, a drug is administered to an organism in the same manner as that described above. In addition, an additional effect, that is, the prevention of the alteration of the drug upon energization is achieved because the second ion exchange membrane blocks the movement of a drug ion to the electrolyte solution holding portion.

It should be noted that the second ion exchange membrane to be used in the present invention must have a slightly low transport number (for example, a transport number of 0.7 to 0.95) because the first electrolytic ion cannot transfer to the first ion exchange membrane in order to substitute the drug ion when the transport number of the second ion exchange membrane is 1. However, even the use of the second ion exchange membrane having such low transport number can sufficiently prevent the transfer of the drug ion to the electrolyte solution holding portion.

The term "transport number" as used herein is defined as a ratio of a charge amount conveyed by the passage of a drug counter ion through the second ion exchange membrane to the total charge conveyed through the second ion exchange membrane when a voltage of the first conductivity type is applied to the side of an electrolyte solution held by the electrolyte solution holding portion in a state where the second ion exchange membrane is placed between the electrolyte solution and a drug solution containing appropriate concentrations of drug ion and drug counter ion (for example, a drug solution used for doping the first ion exchange membrane with the drug ion).

In the invention according to claim 8, the electrolysis of water occurs at an interface between the first and second ion exchange membranes in some cases depending on energization conditions and the like. Therefore, a semi-permeable membrane capable of permitting the passage of at least the first electrolytic ion can be interposed between the first and second ion exchange membranes for preventing the electrolysis.

The second ion exchange membrane in claim 8 can be replaced with a semi-permeable membrane. The same effect as that of the invention according to claim 8 can be achieved by using, as the semi-permeable membrane, a semi-permeable membrane having molecular weight cut-off property with which the passage of the first electrolytic ion can be permitted while the passage of a drug ion is blocked.

The interface between the second ion exchange membrane and the first ion exchange membrane, each interface among the second ion exchange membrane, the semi-permeable membrane, and the first ion exchange membrane, and/or the interface between the semi-permeable membrane and the first ion exchange membrane can be joined integrally by means of, for example, thermocompression bonding. The integral joining can achieve the same effect as that described above with respect to claim 6.

In the invention according to claim 7, it is preferable that: the electrode assembly further include a second ion exchange membrane of the second conductivity type placed on the front surface side of the doping layer; and the electrolyte solution holding portion be placed on the front surface side of the second ion exchange membrane (claim 9) .

In such electrode assembly, a drug is administered to an organism in the same manner as in the invention according to claim 7. In addition, an additional effect, that is, the prevention of the alteration of the drug upon energization is achieved because the second ion exchange membrane blocks the transfer of a drug ion to the doping electrode.

The second ion exchange membrane in claim 9 can be replaced with a semi-permeable membrane. The same effect as that of the invention according to claim 9 can be achieved by using, as the semi-permeable membrane, a semi-permeable membrane having molecular weight cut-off property with which the passage of the first electrolytic ion can be permitted while the passage of a drug ion is blocked.

The interface between the doping electrode and the second ion exchange membrane or the interface between the doping electrode and the semi-permeable membrane can be joined integrally in the same manner as that described above with respect to claim 6. The integral joining can achieve the same effect as that described above with respect to claim 6.

In the invention according to any one of claims 1 to 3, it is preferable that: the electrode assembly further include a first ion exchange membrane of the first conductivity type that is placed on the front surface side of the doping layer and that is doped with a drug ion of the first conductivity type; and the doping layer be doped with an ion of the first conductivity type (claim 10).

Such electrode assembly can be used as a working electrode assembly in an iontophoresis device. A voltage of the first conductivity type is applied to the doping electrode in a state where the first ion exchange membrane is brought into contact with the skin of an organism, whereby an ion of the first conductivity type in the doping layer transfers to the first ion exchange membrane, and a drug ion in the first ion exchange membrane substituted by the ion transfers into the organism.

In this case, energization from the doping electrode to the first ion exchange membrane is caused by the transfer of an ion of the first conductivity type in the doping layer to the first ion exchange membrane. Therefore, the generation of the above-described gas or unpreferable ion can be suppressed.

In the present invention, a drug ion is administered to an organism from the ion exchange membrane of the first conductivity type doped with the drug ion. Accordingly, the same effects as that of the invention according to claim 7 such as an increase in efficiency of administration of a drug and an improvement of the stability of the drug ion are achieved.

In addition, in the present invention, the doping layer is doped with an ion of the first conductivity type for substituting a drug ion. Therefore, the electrolyte solution holding portion in the invention according to claim 7 can be omitted, so the need for handling a wet member can be completely eliminated upon assembly of the electrode assembly. Furthermore, the assembly of the electrode assembly requires only two members: the doping electrode and the first ion exchange membrane. Accordingly, in the present invention, the assembly work of the electrode assembly is extremely simplified. In addition, the automation of the production of the electrode assembly and the mass production of the electrode assembly can be extremely easily performed, or the production cost of the electrode assembly can be significantly reduced.

In the present invention, the doping layer can be doped with an ion of the first conductivity type in the same manner as that described above with respect to claim 4, and the first ion exchange membrane can be doped with a drug ion in the same manner as that described above with respect to claim 7.

The ion of the first conductivity type with which the doping layer is to be doped is preferably an ion having a mobility comparable to or smaller than that of the drug ion owing to the same reason as that described above with respect to claim 7.

The interface between the doping electrode and the first ion exchange membrane can be joined integrally by means of, for example, thermocompression bonding. The integral joining can achieve the same effect as that described above with respect to claim 6.

In the invention according to claim 10, it is preferable that: the electrode assembly further include a second ion exchange membrane of the second conductivity type placed on the front surface side of the doping layer; and the first ion exchange membrane be placed on the front surface side of the second ion exchange membrane (claim 11).

In such electrode assembly, the same effect as that of the invention according to claim 10 is achieved. In addition, an additional effect, that is, the prevention of the alteration of the drug during energization is achieved because the second ion exchange membrane blocks the transfer of a drug ion to the doping layer.

It should be noted that the second ion exchange membrane to be used in the present invention has a slightly low transport number (for example, a transport number of 0.7 to 0.95) in the same manner as in claim 7 owing to the same reason as that described above with respect to claim 7.

The second ion exchange membrane in the invention according to claim 10 can be replaced with a semi-permeable membrane. The same effect as that of the invention according to claim 10 can be achieved by using, as the semi-permeable membrane, a semi-permeable membrane having molecular weight cut-off property with which the passage of the first electrolytic ion can be permitted while the passage of a drug ion is blocked.

The interface between the doping electrode and the second ion exchange membrane or the semi-permeable membrane and/or the interface between the second ion exchange membrane or the semi-permeable membrane and the first ion exchange membrane can be joined integrally by means of, for example, thermocompression bonding. The integral joining can achieve the same effect as that described above with respect to claim 6.

In the invention according to any one of claims 1 to 3, the doping layer can be doped with a drug ion of the first conductivity type (claim 12).

Such electrode assembly can be used as a working electrode assembly in an iontophoresis device. A drug ion in the doping layer can be administered to an organism by applying a voltage of the first conductivity type to the doping electrode in a state where the doping layer is brought into contact with the skin of the organism.

In this case, energization from the doping electrode to the skin of the organism is caused by the de-doping of the drug ion with which the doping layer is doped to transfer to the organism. Therefore, the generation of the above-described gas or unpreferable ion can be suppressed.

With such constitution, a working electrode assembly can be constituted by a single member (the doping electrode). Accordingly, a production step can be significantly simplified, and the mass production and a reduction in production cost can be easily realized.

The doping layer doped with a drug ion of the first conductivity type functions as an ion exchange membrane of the first conductivity type. Therefore, the transfer of an organism counter ion to the doping layer upon administration of a drug is blocked, so excellent property can be obtained in terms of efficiency of administration of a drug.

The doping layer can be doped with a drug ion through energization as a result of applying a voltage of the second conductivity type to the doping electrode in a state where the doping layer is immersed in a drug solution containing an appropriate concentration of drug ion.

In the invention according to any one of claims 1 to 3, the electrode assembly can further include a first ion exchange membrane of the first conductivity type placed on the front surface side of the doping layer (claim 13).

Such electrode assembly can be used as a working electrode assembly in an iontophoresis device by doping the first ion exchange membrane, or the first ion exchange membrane and the doping layer, with a drug ion. The drug ion with which the first ion exchange membrane is, or the first ion exchange membrane and the doping layer are doped can be administered to an organism by applying a voltage of the first conductivity type to the doping electrode in a state where the first ion exchange membrane is brought into contact with the skin of the organism.

The first ion exchange membrane can be doped with a drug ion through energization as a result of applying a voltage of the second conductivity type to the doping electrode in a state where the doping layer is immersed in a drug solution containing an appropriate concentration of drug ion.

In this case, a plus ion bound to an ion exchange group in the first ion exchange membrane and substituted by the drug ion from the drug solution transfers to the doping layer, so the layer is doped with the ion. Alternatively, the doping layer is doped with the drug ion of the drug solution as well depending on conditions for performing the doping.

Energization from the doping electrode to the first ion exchange membrane upon administration of a drug is caused by the transfer of the plus ion or drug ion with which the doping layer is doped as described above to the first ion exchange membrane. Therefore, the generation of the above-described gas or unpreferable ion can be prevented. The drug ion with which the first ion exchange membrane is doped is substituted by an ion transferring from the doping layer, to thereby transfer to an organism.

In the present invention, the efficiency of administration of a drug can be increased because the first ion exchange membrane blocks the transfer of an organism counter ion to the doping layer.

Furthermore, in the present invention, unlike the invention according to claim 12, no constitution is adopted, in which the doping layer is brought into direct contact with the skin of an organism. Therefore, a drug can be safely administered even when a doping layer which is not preferably brought into contact with the skin of an organism is used.

In the present invention, the electrode assembly is composed only of two members: the doping electrode and the first ion exchange membrane. In addition, there is no need to handle a wet member upon assembly of a working electrode assembly. Therefore, in the present invention, the assembly work of the electrode assembly is extremely simplified. As a result, the automation of the production of the electrode assembly and the mass production of the electrode assembly can be extremely easily performed, or the production cost of the electrode assembly can be significantly reduced.

The doping of the first ion exchange membrane with the drug ion may be performed at any time during the period commencing on the stage of the production of an iontophoresis device and ending on the stage immediately before use (the administration of a drug to an organism).

The interface between the doping electrode and the first ion exchange membrane can be joined integrally by means of, for example, thermocompression bonding. The integral joining can achieve the same effect as that described above with respect to claim 6.

According to another aspect of the present invention, there is provided an iontophoresis device including: a working electrode assembly holding a drug ion of a first conductivity type; and a non-working electrode assembly as a counter electrode of the working electrode assembly, characterized in that the non-working electrode assembly includes an electrode in which a doping layer made of a substance effecting an electrochemical reaction owing to the doping or de-doping of an ion is formed (claim 14).

In such iontophoresis device, a voltage of the second conductivity type is applied to the doping electrode of the non-working electrode assembly upon administration of a drug, but the generation of: a gas such as a hydrogen gas, an oxygen gas, or a chlorine gas; or an unpreferable ion such as a hydrogen ion, a hydroxide ion, or hypochlorous acid at the non-working electrode assembly at this time can be prevented.

That is, when the doping layer is not doped with any ion of the second conductivity type, energization in the non-working electrode assembly is caused by the transfer of an ion of the first conductivity type on the skin of an organism or in the organism to the doping layer such that the layer is doped with the ion. When the doping layer is doped with an ion of the second conductivity type, the energization is caused by the de-doping of the ion of the second conductivity type from the doping layer to transfer to the side of the organism in addition to the doping of the doping layer with the ion of the first conductivity type.

The working electrode assembly in the present invention may hold a drug ion at a drug solution holding portion holding a drug solution like the invention according to claim 4 or the like. Alternatively, the working electrode assembly may hold the drug ion with which the first ion exchange membrane or the doping layer is doped like the invention according to claim 7, 10, 12, or the like. In addition, the working electrode assembly of the present invention is not necessarily need to have a doping electrode.

In the invention according to claim 14, the non-working electrode assembly preferably further includes a third ion exchange membrane of the first conductivity type placed on the front surface side of the doping layer (claim 15).

In such electrode assembly, energization is performed in a state where the third ion exchange membrane is brought into contact with the skin of an organism. Therefore, an iontophoresis device capable of administering a drug ion without bringing the doping layer into direct contact with the skin is realized.

It should be noted that energization in the non-working electrode assembly is mainly caused by the transfer of an ion of the first conductivity type on the skin of an organism or in the organism to the doping layer such that the layer is doped with the ion.

In the invention according to claim 14, it is preferable that: the no-working electrode assembly further include a third ion exchange membrane of the second conductivity type that is placed on the front surface side of the doping layer; and the doping layer be doped with an ion of the second conductivity type (claim 16).

Such electrode assembly realizes an iontophoresis device capable of administering a drug ion without bringing the doping layer into direct contact with a skin in the same manner as in the invention according to claim 15.

It should be noted that energization in the non-working electrode assembly is mainly caused by the de-doping of an ion of the second conductivity type from the doping layer to transfer to the side of an organism.

The interface between the doping electrode and the third ion exchange membrane in the invention according to claim 15 or 16 can be joined integrally by means of, for example, thermocompression bonding. The integral joining can achieve the same effect as that described above with respect to claim 6.

In the invention according to claim 14, the non-working electrode assembly can further include a second electrolyte solution holding portion holding an electrolyte solution, the second electrolyte solution holding portion being placed on the front surface side of the doping layer. In this case, energization is caused by, for example, the transfer of the first electrolytic ion in the second electrolyte solution holding portion to the doping layer such that the layer is doped with the ion and the transfer of the second electrolytic ion to an organism.

It is preferable that: the doping electrode according to any one of claims 1 to 16 further include a conductive base material; and the doping layer be stacked on the conductive base material (claim 17).

As described above, the conductivity of the doping layer can be improved by doping the layer with an ion as an electron acceptor or an electron donor. Alternatively, an iontophoresis device capable of administering a drug with improved efficiency can be realized by placing the doping layer on the conductive base material to reduce the surface resistance of the doping electrode in such a manner that energization can be performed from the doping layer at a uniform current density.

The doping layer can be formed on the conductive base material by means of, for example, a method involving: applying, to the conductive base material, a powder-like conductive polymer blended with an appropriate polymer binder or a solution of a conductive polymer in an appropriate polar organic solvent; and subjecting the resultant to curing, solvent removal, or the like, or a method involving: immersing the conductive base material in a solution of a monomer for producing a conductive polymer; and performing electrolytic polymerization.

In the invention according to claim 17, the conductive base material is preferably a conductive sheet made of a carbon fiber or carbon fiber paper (claim 18).

In this case, the doping electrode can be formed without using a metallic member. As a result, a metal ion eluted from such metallic member can be prevented from transferring to an organism to do harm to the health. In addition, energization can occur from the doping electrode at a uniform current density because the carbon fiber or carbon fiber paper is a material having a low surface resistance. An iontophoresis device including an electrode assembly having enough flexibility to follow the irregularities of the skin of an organism or the movement of the organism can be provided because the carbon fiber or carbon fiber paper is a material having high flexibility.

In this case, an electrode described in JP 2004-317317 A or JP 2005-222892 A by the applicant of the present invention can be used.

That is, in the invention according to claim 18, the electrode can further include a terminal member with carbon mixed in a polymer matrix, the terminal member being attached to the conductive sheet (claim 19). Alternatively, the electrode can further include an extension portion that is formed integrally with the conductive sheet and that is made of a carbon fiber or carbon fiber paper.

The term "drug" as used herein refers to a substance which may be or may not be prepared, which has a certain drug effect or pharmacological effect, and which is applicable to an organism for purposes including the therapy, recovery, and prevention of a disease and the promotion and maintenance of the health.

The term "drug ion" as used herein refers to an ion which is produced by the dissociation of a drug to ions and which is responsible for a drug effect or a pharmacological action, and the term "drug counter ion" as used herein refers to a counter ion of the drug ion. The dissociation of the drug to a drug ion may occur as a result of the dissolution of the drug into a solvent such as water, an alcohol, an acid, or an alkali, or may occur as a result of, for example, the application of a voltage or the addition of an ionizing agent.

The term "skin" as used herein refers to the surface of an organism to which a drug can be administered by iontophoresis, and includes a mucosa in an oral cavity. The term "organism" as used herein refers to a human being or an animal.

The term "first conductivity type" as used herein refers to plus or minus electrical polarity, and the term "second conductivity type" as used herein refers to the electrical polarity (minus or plus) opposite to the first conductivity type.

Each of the first electrolytic ion and the second electrolytic ion in the electrolyte solution of the electrolyte solution holding portion in the present invention is not needed to be of a single kind, and one or both of the ions may be of multiple kinds. Similarly, each of the drug ion in the drug solution holding portion and the drug ion with which the first ion exchange membrane or the doping layer is doped is not needed to be of a single kind, and may be of multiple kinds.

Known examples of an ion exchange membrane include various ion exchange membranes such as (1) a heterogenenous ion exchange membrane obtained by: dispersing an ion exchange resin into a binder polymer; and forming the resultant into a film through, for example, molding under heat and (2) a homogeneous ion exchange membrane obtained by: impregnating and filling a base material such as cloth, a net, or a porous film with a solution prepared by dissolving a composition composed of a monomer, a cross-linkable monomer, a polymerization initiator, or the like into which an ion exchange group can be introduced or a resin having a functional group into which an ion exchange group can be introduced into a solvent; subjecting the resultant to polymerization or solvent removal; and subjecting the resultant to a treatment for introducing an ion exchange group as well as an ion exchange resin formed into a membrane shape. Any one of those arbitrary ion exchange membranes can be used for the ion exchange membrane of the present invention. Of those, an ion exchange membrane of a type in which the pores of a porous film are filled with an ion exchange resin is particularly preferably used.

More specifically, an ion exchange membrane into which a cation exchange group is introduced such as a NEOSEPTA (CM-1, CM-2, CMX, CMS, or CMB) manufactured by Tokuyama Co., Ltd can be used for the cation exchange membrane. An ion exchange membrane into which an anion exchange group is introduced such as a NEOSEPTA (AM-1, AM-3, AMX, AHA, ACH, or ACS) manufactured by Tokuyama Co., Ltd can be used for the anion exchange membrane.

The term "ion exchange membrane of the first conductivity type" as used herein refers to an ion exchange membrane having a function of selectively passing an ion of the first conductivity type. That is, the "ion exchange membrane of the first conductivity type" is a cation exchange membrane when the first conductivity type is plus, while the "ion exchange membrane of the first conductivity type" is an anion exchange membrane when the first conductivity type is minus.

Similarly, the term "ion exchange membrane of the second conductivity type" as used herein refers to an ion exchange membrane having a function of selectively passing an ion of the second conductivity type. That is, the "ion exchange membrane of the second conductivity type" is a cation exchange membrane when the second conductivity type is plus, while the "ion exchange membrane of the second conductivity type" is an anion exchange membrane when the second conductivity type is minus.

Examples of a cation exchange group to be introduced into the cation exchange membrane include a sulfonic group, a carboxylic group, and a phosphoric group. The transport number of an ion exchange membrane can be controlled depending on the kind of a cation exchange group to be introduced. For example, the use of a sulfonic group as a strong acidic group provides a cation exchange membrane having a high transport number.

Examples of an anion exchange group to be introduced into the anion exchange membrane include a primary amino group, a secondary amino group, a tertiary amino group, a quaternary ammonium group, a pyridyl group, an imidazole group, a quaternary pyridinium group, and a quaternary imidazolium group. The transport number of an ion exchange membrane can be controlled depending on the kind of an anion exchange group to be introduced. For example, the use of a quaternary ammonium group or a quaternary pyridinium group as a strong basic group provides an anion exchange membrane having a high transport number.

Known examples of a treatment for introducing a cation exchange group include various approaches such as sulfonation, chlorosulfonation, phosphonation, and hydrolysis. Known examples of a treatment for introducing an anion exchange group include various approaches such as amination and alkylation. The transport number of an ion exchange membrane can be adjusted by adjusting conditions under which a treatment for introducing an ion exchange group is performed.

In addition, the transport number of an ion exchange membrane can be adjusted depending on, for example, the amount of an ion exchange resin in the ion exchange membrane and the pore size of the membrane. For example, in the case of an ion exchange membrane of a type in which a porous film is filled with an ion exchange resin, an ion exchange membrane obtained by filling a porous film with an ion exchange resin at a filling ratio of preferably 5 to 95 mass%, more preferably 10 to 90 mass%, or particularly preferably 20 to 60 mass% can be used, the porous film having formed thereon a large number of small pores having a mean pore size of preferably 0.005 to 5.0 µm, more preferably 0.01 to 2.0 µm, or most preferably 0.02 to 0.2 µm (a mean flow pore size measured in conformance with the bubble point method (JIS K3832-1990)) at a porosity of preferably 20 to 95%, more preferably 30 to 90%, or most preferably 30 to 60% and having a thickness of preferably 5 to 140 µm, more preferably 10 to 120 µm, or most preferably 15 to 55 µm. The transport number of the ion exchange membrane can be adjusted depending also on the mean pore size of the small pores and the porosity of the porous film, and the filling ratio of the ion exchange resin.

The term "blocking of the passage of an ion" to be described for an ion exchange membrane of the first conductivity type or the second conductivity type in the specification does not always mean the blocking of the passage of all ions. The term includes the case where, even when the passage of an ion occurs with a certain speed, the degree of the passage is so small that the passage of a drug ion is suppressed to the extent that no alteration of a drug occurs near an electrode even if the device is stored over a practically sufficient period, or the passage of an organism counter ion is suppressed to the extent that the efficiency of administration of the drug can be sufficiently increased.

Similarly, the term "permission of the passage of an ion" to be described for an ion exchange membrane of the first conductivity type or the second conductivity type in the specification does not mean that no restrictions are imposed on the passage of an ion. The term includes the case where an ion is allowed to pass with a sufficiently high speed or amount as compared to an ion having a conductivity type opposite to that of the former ion even when the passage of the ion is restricted to some extent.

The terms "blocking of the passage of an ion" and "permission of the passage of an ion" to be described for a semi-permeable membrane in the specification have the same meanings as those described above, and do not mean the blocking of the passage of all ions or that no restrictions are imposed on the passage of an ion.

### BREIF DESCRIPTION OF THE DRAWINGS

[Fig. 1] An explanatory view showing the schematic constitution of an iontophoresis device according to an embodiment of the present invention.
[Figs. 2] Figs. 2(A) to 2(D) are explanatory sectional views each showing the constitution of a working electrode assembly of an iontophoresis device according to an embodiment of the present invention.
[Figs. 3] Figs. 3(A) to 3(D) are explanatory sectional views each showing the constitution of a working electrode assembly of an iontophoresis device according to an embodiment of the present invention.
[Figs. 4] Figs. 4(A) and 4(B) are explanatory sectional views each showing the constitution of a working electrode assembly of an iontophoresis device according to an embodiment of the present invention.
[Figs. 5] Figs. 5(A) and 5(B) are explanatory sectional views each showing the constitution of a working electrode assembly of an iontophoresis device according to an embodiment of the present invention.
[Figs. 6] Figs. 6(A) to 6(D) are explanatory sectional views each showing the constitution of a non-working electrode assembly of an iontophoresis device according to an embodiment of the present invention.
[Figs. 7] Fig. 7(A) is a plan view of an electrode to be used for an iontophoresis device according to an embodiment of the present invention, Fig. 7(B) is a sectional view taken along the line A-A of Fig. 7 (A), and Fig. 7 (C) is a sectional view showing a modification of Fig. 7(B).
[Figs. 8] Fig. 8 (A) is a plan view of an electrode according to another aspect to be used for an iontophoresis device according to an embodiment of the present invention, Fig. 8 (B) is a sectional view taken along the line A-A of Fig. 8(A), and Fig. 8(C) is a sectional view showing a state where the electrode is housed in a container.
[Fig. 9] An explanatory view showing the constitution of a conventional iontophoresis device.
[Fig. 10] An explanatory view showing the constitution of another conventional iontophoresis device.

### BEST MODE FOR CARRYING OUT THE INVENTION

Hereinafter, an embodiment of the present invention will be described with reference to the drawings.

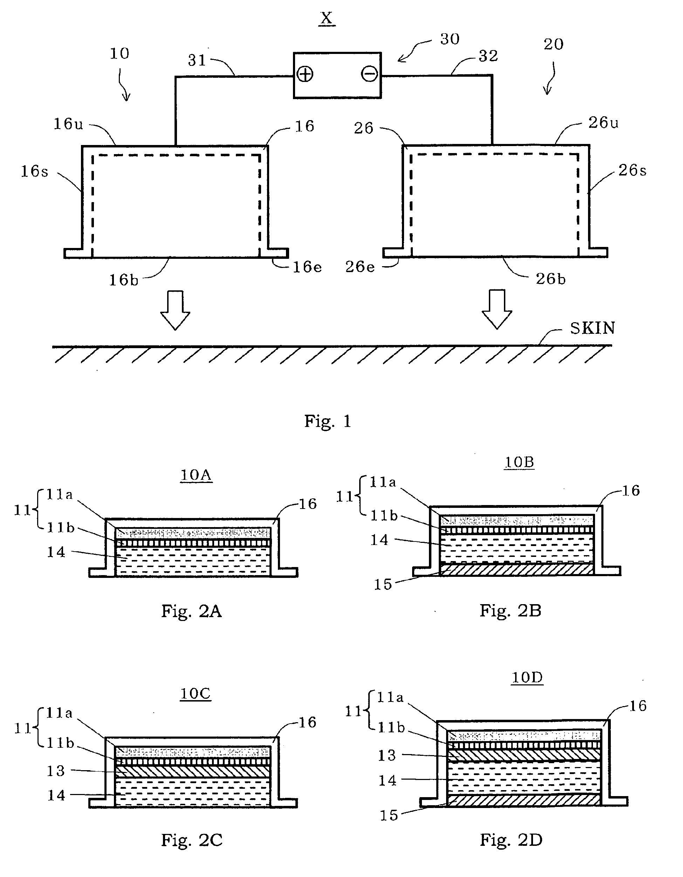

Fig. 1 is an explanatory view showing the schematic constitution of an iontophoresis device X according to the present invention.

In the following, for convenience of description, an iontophoresis device for administering a drug whose drug component dissociates to plus drug ions (for example, lidocaine hydrochloride or morphine hydrochloride) will be exemplified. An iontophoresis device for administering a drug whose drug component dissociates to minus ions (for example, ascorbic acid), the device being capable of achieving substantially the same effect as that of the following embodiment, can be constituted by reversing the polarity of an electric power source, the conductivity type of each ion exchange membrane, and the conductivity type of an ion with which a doping layer or a cation exchange membrane is doped in the following description.

As shown in the figure, the iontophoresis device X includes: an electric power source 30; a working electrode assembly 10 connected to the plus pole of the electric power source 30 via an electric supply line 31; and a non-working electrode assembly 20 connected to the minus pole of the electric power source 30 via an electric supply line 32.

The working electrode assembly 10/the non-working electrode assembly 20 is a container 16/26 composed of an upper wall 16u/26u and an outer peripheral wall 16s/26s. A space capable of housing various structures to be described later is formed in the container 16/26, and a lower surface 16b/26b of the container 16/26 is opened.

The container 16 or 26 can be formed of an arbitrary material such as a plastic, but is preferably formed of a flexible material capable of: preventing the evaporation of water from the inside of the container and the penetration of foreign matter from the outside; and following the irregularities of the skin of an organism or the movement of the organism. In addition, a removable liner composed of an appropriate material for preventing the evaporation of water and the mixing of foreign matter during storage of the iontophoresis device X can be stuck to the lower surface 16b/26b of the container 16/26. An adhesive layer for improving adhesiveness to a skin upon administration of a drug can be arranged on a lower end portion 16e/26e of the outer peripheral wall 16s/26s.

The container 16 or 26 is not necessarily arranged in the absence of a wet member such as a drug solution holding portion or an electrolyte solution holding portion (a member with a high water content) like working electrode assemblies 10H to 10K and non-working electrode assemblies 20A to 20C to be described later.

A battery, a constant voltage device, a constant current device, a constant voltage/current device, or the like can be used as the electric power source 30. It is preferable to use a constant current device whose current can be adjusted in the range of 0.01 to 1.0 mA/cm², or preferably 0.01 to 0.5 mA/cm², and which operates under safe voltage conditions, specifically at 50 V or less, or preferably 30 V or less.

Figs. 2(A) to 2(D) are explanatory sectional views showing the constitutions of working electrode assemblies 10A to 10D each of which can be used as the working electrode assembly 10 of the iontophoresis device X.

The working electrode assembly 10A includes: an electrode 11 having a conductive base material 11a connected to the electric supply line 31 and a doping layer 11b formed on one surface of the base material 11a and composed of polyaniline; and a drug solution holding portion 14 holding a drug solution in contact with the doping layer 11b.

The electrode 11 may include, for example, the base material 11a formed of a carbon sheet and the doping layer 11b formed by applying a polyaniline solution in which a polyaniline salt is mixed in an NMP (N-methylpyrrolidone) solution containing PVDF (polyvinylidene fluoride) onto the base material 11a followed by drying.

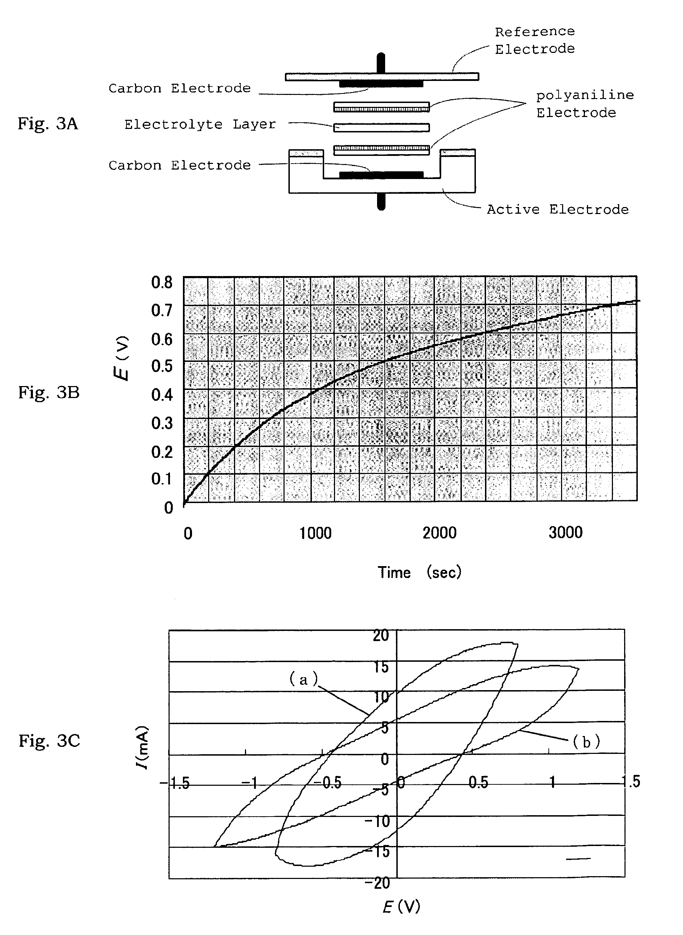

200 mg of polyaniline solution containing the polyaniline salt, PVDF, and NMP at the weight ratio of 1: 1: 9, in which the polyaniline salt was prepared by adding 1-N hydrochloric acid to polyaniline-emeraldine base followed by filtration and drying, were applied onto the carbon sheet having the thickness of 300 µm and diameter of 17 mm. The resultant was dried in vacuum at 100°C for 1 hour to produce the electrode 11 experimentally. The chronopotentiometry and the measurement of cyclic voltammogram were performed using the measurement cell shown in Fig. 3(A).

Fig. 3(B) shows the measurement result for the capacitor capacitance of electrode 11 under the constant current condition of 0.3 mA/cm2 by chronopotentiometry. It was confirmed that the electrode 11 had extremely large capacitor capacitance.

Fig. 3(C) shows the measurement results of the cyclic voltammogram (a) in the case where a non-woven fabric immersed with a solution containing 0.9% NaCl and 2% HPC (hydroxypropyl cellulose) was used as a electrolyte solution layer in the measurement cell and (b) in the case where a non-woven fabric immersed with a solution containing 10% lidocaine hydrochloride and 2% HPC was used as a electrolyte solution layer in the measurement cell. Note that the range of potential sweep was set to -1.2 to +1.2 V in the case of (a) and the range of potential sweep was set to -0.8 to +0.8 V in the case of (b), and the potential sweep rate was set to 10 mV/sec in the both cases of (a) and (b) to perform the measurement. Fig. 3 (C) shows that the electrode 11 is excellent in charge and discharge properties and has high resistance to the deterioration and the like caused by the oxidation-reduction cycle.

[0148] The drug solution is a solution of a drug whose drug component dissociates to plus drug ions. The drug solution holding portion 14 can hold the drug solution in a liquid state, or can hold the drug solution by impregnating an absorbing carrier such as a gauze, filter paper, or a gel with the drug solution.

[0149] In the working electrode assembly 10A, a plus voltage is applied to the electrode 11 in a state where the drug solution holding portion 14 is brought into contact with the skin of an organism, whereby a drug ion in the drug solution holding portion 14 is administered to the organism. In this case, energization from the electrode 11 to the drug solution holding portion 14 is entirely or partially caused by the transfer of a minus ion in the drug solution to the doping layer 11b such that the layer is doped with the ion. Therefore, the generation of an oxygen gas or a chlorine gas or the production of a hydrogen ion or hypochlorous acid due to energization can be prevented or at least reduced.

[0150] The doping layer 11b has a thickness of typically 10 nm to 100 µm, or particularly preferably 1 to 10 µm.

[0151] The working electrode assembly 10B includes: the electrode 11 and the drug solution holding portion 14 identical to those of the working electrode assembly 10A; and further the cation exchange membrane 15 placed on the front surface side of the drug solution holding portion 14.

The working electrode assembly 10B achieves the same effect as that of the working electrode assembly 10A concerning the prevention of: the generation of a gas; or the production of an unpreferable ion upon energization. The working electrode assembly 10B achieves an additional effect, that is, an increase in efficiency of administration of a drug ion because the transfer of an organism counter ion to the drug solution holding portion 14 is blocked by the cation exchange membrane 15.

The working electrode assembly 10C includes: the electrode 11 and the drug solution holding portion 14 identical to those of the working electrode assembly 10A; and an anion exchange membrane 13 placed between the electrode 11 and the drug solution holding portion 14.

In the working electrode assembly 10C, energization from the electrode 11 to the drug solution holding portion 14 is caused by the transfer of a minus ion in the drug solution holding portion 14 to the doping layer 11b via the anion exchange membrane 13 such that the layer is doped with the ion. Therefore, the working electrode assembly 10C achieves the same effect as that of the working electrode assembly 10A concerning the prevention of: the generation of a gas; or the production of an unpreferable ion upon energization.

Furthermore, the working electrode assembly 10C achieves an additional effect, that is, the prevention of the decomposition and alteration of a drug upon energization because the transfer of a drug ion in the drug solution holding portion 14 to the doping layer 11b is blocked by the anion exchange membrane 13.

The working electrode assembly 10D includes: the electrode 11 and the drug solution holding portion 14 identical to those of the working electrode assembly 10A; the anion exchange membrane 13 placed between the electrode 11 and the drug solution holding portion 14; and the cation exchange membrane 15 placed on the front surface side of the drug solution holding portion 14.

Therefore, the working electrode assembly 10D achieves the same effect as that of the working electrode assembly 10A concerning the prevention of: the generation of a gas; or the production of an unpreferable ion upon energization. The working electrode assembly 10D achieves additional effects, that is, the prevention of the decomposition and alteration of a drug upon energization and an increase in efficiency of administration of the drug as in the case of the working electrode assemblies 10B and 10C.

In each of the working electrode assemblies 10C and 10D, the electrode 11 and the anion exchange membrane 13 can be joined and integrated with each other by means of an approach such as thermocompression bonding. This action can improve a state of energization from the electrode 11 to the anion exchange membrane 13 or simplify the assembly work of each of the working electrode assemblies 10C and 10D.

Figs. 3(A) to 3(C) are explanatory sectional views showing the constitutions of working electrode assemblies 10E to 10G according to still another aspect each of which can be used as the working electrode assembly 10 of the iontophoresis device X.

The working electrode assembly 10E includes: the electrode 11 identical to that of the working electrode assembly 10A; an electrolyte solution holding portion 12 holding an electrolyte solution in contact with the doping layer 11b; and the cation exchange membrane 15 placed on the front surface side of the electrolyte solution holding portion 12 and doped with a plus drug ion.

In the working electrode assembly 10E, a plus voltage is applied to the electrode 11 in a state where the cation exchange membrane 15 is brought into contact with the skin of an organism, whereby the drug ion with which the cation exchange membrane 15 is doped is administered to the organism.

In this case, a drug can be administered with high efficiency because the cation exchange membrane 15 blocks the transfer of an organism counter ion to the electrolyte solution holding portion 12.

In addition, energization from the electrode 11 to the electrolyte solution holding portion 12 is entirely or partially caused by the transfer of a minus ion in the electrolyte solution to the doping layer 11b such that the layer is doped with the ion. Therefore, the generation of an oxygen gas or a chlorine gas or the production of a hydrogen ion or hypochlorous acid due to energization can be prevented or at least reduced.

Energization from the electrolyte solution holding portion 12 to the cation exchange membrane 15 is caused by the transfer of a plus ion in the electrolyte solution holding portion 12 to the cation exchange membrane 15. The plus ion is substituted by a drug ion that has transferred to an organism, to thereby bind to an ion exchange group in the cation exchange membrane 15.

The electrolyte solution holding portion 12 of the working electrode assembly 10E may hold the electrolyte solution in a liquid state, or may hold the electrolyte solution by impregnating an absorbing carrier such as a gauze, filter paper, or a gel with the electrolyte solution.

When a plus ion in the electrolyte solution holding portion 12 has a mobility larger than that of a drug ion, the transfer of the plus ion to an organism preferentially may occur, so the efficiency of administration of a drug may reduce. Therefore, the electrolyte solution of the electrolyte solution holding portion 12 preferably has a constitution free of a plus ion having a mobility comparable to or larger than that of a drug ion.

The cation exchange membrane 15 can be doped with a drug ion by immersing the cation exchange membrane 15 in a drug solution containing an appropriate concentration of the drug ion.

The working electrode assembly 10F includes: the electrode 11, the electrolyte solution holding portion 12, and the cation exchange membrane 15 identical to those of the working electrode assembly 10E; and further the anion exchange membrane 13 placed between the electrolyte solution holding portion 12 and the cation exchange membrane 15.

The working electrode assembly 10F achieves the same effect as that of the working electrode assembly 10E concerning the prevention of: the generation of a gas; or the production of an unpreferable ion upon energization. The working electrode assembly 10F achieves an additional effect, that is, the prevention of the alteration of a drug near the electrode 11 upon energization because the transfer of the drug ion with which the cation exchange membrane 15 is doped to the electrolyte solution holding portion 12 is blocked by the anion exchange membrane 13.

Causing energization from the electrolyte solution holding portion 12 to the cation exchange membrane 15 in the working electrode assembly 10F requires that a plus ion in the electrolyte solution holding portion 12 should pass through the anion exchange membrane 13 to transfer to the cation exchange membrane 15. Therefore, an anion exchange membrane having a slightly low transport number is used for the anion exchange membrane 13.

In the working electrode assembly 10F, the electrolysis of water occurs at an interface between the anion exchange membrane 13 and the cation exchange membrane 15 in some cases depending on energization conditions and the like. Therefore, a semi-permeable membrane capable of permitting the passage of at least a plus ion in the electrolyte solution holding portion 12 can be further placed between the anion exchange membrane 13 and the cation exchange membrane 15 for preventing the electrolysis. The interface between the anion exchange membrane 13 and the cation exchange membrane 15 or each interface among the anion exchange membrane 13, the semi-permeable membrane, and the cation exchange membrane 15 can be joined by means of an approach such as thermocompression bonding. This action can improve energization property between them and handleability thereof.

The anion exchange membrane 13 in the working electrode assembly 10F permits the passage of a plus ion in the electrolyte solution holding portion 12. Meanwhile, the same effect as that described above can be obtained even when the membrane is replaced with a semi-permeable membrane capable of blocking the passage of a drug ion.

The working electrode assembly 10G includes: the electrode 11, the electrolyte solution holding portion 12, and the cation exchange membrane 15 identical to those of the working electrode assembly 10E; and the anion exchange membrane 13 placed between the electrode 11 and the electrolyte solution holding portion 12.

In the working electrode assembly 10G, energization from the electrode 11 to the electrolyte solution holding portion 12 is caused by the transfer of a minus ion in the electrolyte solution holding portion 12 to the doping layer via the anion exchange membrane such that the layer is doped with the ion. Therefore, the working electrode assembly 10G achieves the same effect as that of the working electrode assembly 10E concerning the prevention of: the generation of a gas; or the production of an unpreferable ion upon energization.

Energization from the electrolyte solution holding portion 12 to the cation exchange membrane 15 occurs in the same manner as in the case of the working electrode assembly 10E. Furthermore, an additional effect, that is, the prevention of the decomposition and alteration of a drug upon energization is achieved because the transfer of the drug ion with which the cation exchange membrane 15 is doped to the doping layer 11b is blocked by the anion exchange membrane 13.

The electrode 11 and the anion exchange membrane 13 are joined and integrated with each other by means of an approach such as thermocompression bonding, whereby energization property between them and the handleability of them can be improved.

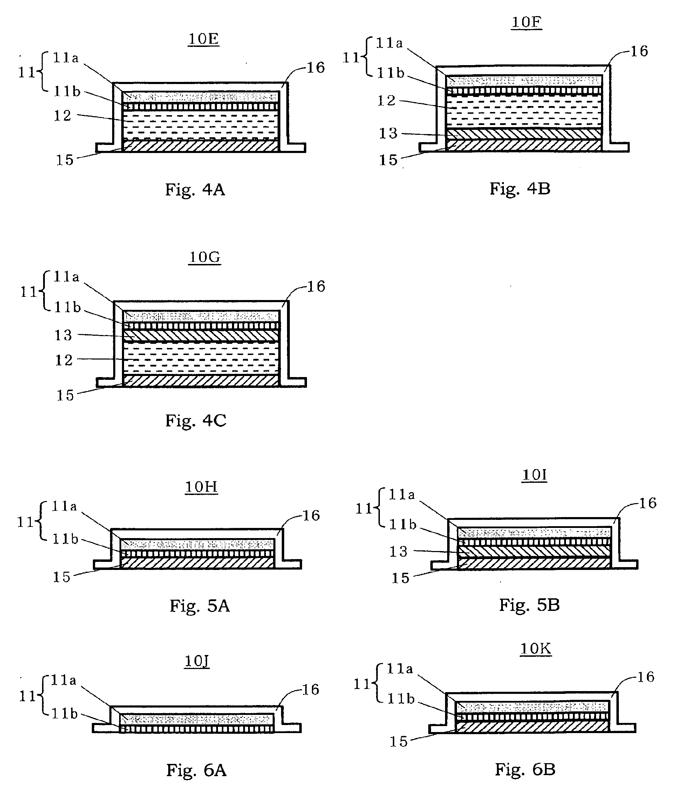

Figs. 4 (A) and 4(B) are explanatory sectional views showing the constitutions of working electrode assemblies 10H and 10I according to still another aspect each of which can be used as the working electrode assembly 10 of the iontophoresis device X.

The working electrode assembly 10H includes: the electrode 11 having the conductive base material 11a connected to the electric supply line 31 and the doping layer 11b formed on one surface of the base material 11a and doped with a plus ion; and the cation exchange membrane 15 placed on the front surface side of the doping layer 11b and doped with a drug ion.

In the working electrode assembly 10H, a plus voltage is applied to the electrode 11 in a state where the cation exchange membrane 15 is brought into contact with the skin of an organism, whereby the drug ion with which the cation exchange membrane 15 is doped is administered to the organism. As a result, a drug can be administered with high efficiency in the same manner as in the case of the working electrode assembly 10E.

In the working electrode assembly 10H, energization from the electrode 11 to the cation exchange membrane 15 is caused by the transfer of the plus ion with which the doping layer 11b is doped to the cation exchange membrane 15. Therefore, the generation of an oxygen gas or a chlorine gas or the production of a hydrogen ion or hypochlorous acid due to energization is prevented or at least reduced. The plus ion that has transferred from the doping layer 11b to the cation exchange membrane 15 is substituted by a drug ion that has transferred to an organism, to thereby bind to an ion exchange group in the cation exchange membrane 15.

As shown in the figure, the working electrode assembly 10H has an extremely simple structure composed only of the electrode 11 and the cation exchange membrane 15, and there is no need to handle a wet member upon assembly of the working electrode assembly 10H. Therefore, the automation of the production of the working electrode assembly 10H and the mass production of the working electrode assembly 10H can be extremely easily performed, and the production cost of the working electrode assembly 10 can be significantly reduced.

The electrode 11 and the cation exchange membrane 15 are joined and integrated with each other by means of an approach such as thermocompression bonding, whereby energization property between them and the handleability of them can be improved.

The doping layer 11b of the working electrode assembly 10H can be doped with a plus ion through energization with the electrode 11 as a minus pole in a state where the doping layer 11b is immersed in an appropriate electrolyte solution. In addition, the cation exchange membrane 15 can be doped with a drug ion in the same manner as that described above with respect to the working electrode assembly 10E.

The doping layer 11b is preferably doped with a plus ion having a mobility smaller than that of a drug ion owing to the same reason as that described above with respect to the working electrode assembly 10E. The plus ion can be a drug ion identical to or different from the drug ion with which the cation exchange membrane 15 is doped.

The working electrode assembly 10I includes: the electrode 11 and the cation exchange membrane identical to those of the working electrode assembly 10H; and the anion exchange membrane 13 placed between the electrode 11 and the cation exchange membrane 15.

In the working electrode assembly 10I, as in the case of the working electrode assembly 10H, the generation of an oxygen gas or a chlorine gas or the production of a hydrogen ion or hypochlorous acid upon administration of a drug is prevented, and there is no need to handle a wet member upon assembly. In addition, an additional effect, that is, the prevention of the decomposition and alteration of a drug upon energization is achieved because the transfer of the drug ion with which the cation exchange membrane 15 is doped to the electrolyte solution holding portion 12 is blocked by the anion exchange membrane 13.

Causing energization from the electrode 11 to the cation exchange membrane 15 in the working electrode assembly 10I requires that the plus ion with which the doping layer 11b is doped should pass through the anion exchange membrane 13 to transfer to the cation exchange membrane 15. Therefore, an anion exchange membrane having a slightly low transport number is used for the anion exchange membrane 13.

The electrode 11, the anion exchange membrane 13, and the cation exchange membrane are joined and integrated with one another by means of an approach such as thermocompression bonding, whereby energization property among them and the handleability of them can be improved.

The same effect as that described above can be achieved even when the anion exchange membrane 13 in the working electrode assembly 10I is replaced with a semi-permeable membrane that blocks the passage of a drug ion while permitting the passage of a plus ion in the electrolyte solution holding portion 12.

Figs. 5 (A) and 5 (B) are explanatory sectional views showing the constitutions of working electrode assemblies 10J and 10K according to still another aspect each of which can be used as the working electrode assembly 10 of the iontophoresis device X.

The working electrode assembly 10J includes the electrode 11 having the conductive base material 11a connected to the electric supply line 31 and the doping layer 11b formed on one surface of the base material 11a.

In the working electrode assembly 10J, the doping layer 11b is doped with a drug ion, and then a plus voltage is applied to the electrode 11 in a state where the doping layer 11b is brought into contact with the skin of an organism, whereby the drug ion with which the doping layer 11b is doped is administered to the organism.

Energization from the doping layer 11b to the skin of the organism is caused by the movement of the drug ion. Therefore, the generation of an oxygen gas or a chlorine gas or the production of a hydrogen ion or hypochlorous acid due to energization is prevented or at least reduced.

In addition, the doping layer 11b doped with the drug ion as a plus ion has a cation exchange function, so the transfer of an organism counter ion from the side of the skin to the doping layer 11b upon administration of a drug is blocked and the drug can be administered with high efficiency.

As shown in the figure, the working electrode assembly 10J has an extremely simple structure composed only of the electrode 11. Therefore, the automation of the production of the working electrode assembly 10 J and the mass production of the working electrode assembly 10J can be extremely easily performed, and the production cost of the working electrode assembly can be significantly reduced.

The doping layer 11b can be doped with the drug ion through energization with the electrode 11 as a minus pole in a state where the doping layer 11b is immersed in a drug solution containing an appropriate concentration of drug ion. The doping may be performed at the stage of the production of the iontophoresis device X or the working electrode assembly 10J, or may be performed immediately before the administration of a drug.

The working electrode assembly 10K includes: the electrode 11 having the conductive base material 11a connected to the electric supply line 31 and the doping layer 11b formed on one surface of the base material 11a; and the cation exchange membrane 15 placed on the front surface side of the electrode 11.

In the working electrode assembly 10K, the cation exchange membrane 15 is, or the cation exchange membrane 15 and the doping layer 11b are doped with a drug ion, and then a plus voltage is applied to the electrode 11 in a state where the cation exchange membrane 15 is brought into contact with the skin of an organism, whereby the drug ion with which the cation exchange membrane 15 is, or the cation exchange membrane 15 and the doping layer 11b are doped is administered to the organism via the cation exchange membrane 15.

In the working electrode assembly 10K, energization from the electrode 11 to the cation exchange membrane 15 is caused by the transfer of the ion with the doping layer 11b is doped to the cation exchange membrane. Therefore, the generation of an oxygen gas or a chlorine gas or the production of a hydrogen ion or hypochlorous acid due to energization is prevented or at least reduced.

Inaddition, the drug can be administered with high efficiency because the transfer of an organism counter ion from an organism to the doping layer 11b is blocked by the cation exchange membrane 15.

The working electrode assembly 10K has an extremely simple structure composed only of the electrode 11 and the cation exchange membrane 15. Therefore, the automation of the production of the working electrode assembly 10K and the mass production of the working electrode assembly 10K can be extremely easily performed, and the production cost of the working electrode assembly can be significantly reduced.

In addition, the working electrode assembly 10K is structured in such a manner that the doping layer 11b does not directly contact with a skin. Therefore, a drug can be administered with no possibility that harm or the like is done to the health of an organism even when the doping layer 11b made of a substance which is not preferably brought into contact with the organism is used.

The cation exchange membrane 15, or the cation exchange membrane 15 and the doping layer 11b, can be doped with the drug ion through energization with the electrode 11 as a minus pole in a state where the cation exchange membrane 15 is immersed in a drug solution containing an appropriate concentration of drug ion. The doping may be performed at the stage of the production of the iontophoresis device X or the working electrode assembly 10K, or may be performed immediately before the administration of a drug.

The electrode 11 and the cation exchange membrane 15 are joined and integrated with each other by means of an approach such as thermocompression bonding, whereby energization property between them and the handleability of them can be improved.

Figs. 6(A) to 6(D) are explanatory sectional views showing the constitutions of non-working electrode assemblies 20A and 20D each of which can be used as the non-working electrode assembly 20 of the iontophoresis device X.

The non-working electrode assembly 20A includes an electrode 21 having a conductive base material 21a connected to the electric supply line 32 and a doping layer 21b formed on the base material 21a.

In the non-working electrode assembly 20A, when a minus voltage is applied to the electrode 21 in a state where the doping layer 21b is brought into contact with an organism, energization is caused by the transfer of a plus ion from the skin of the organism to the doping layer 21b such that the layer is doped with the ion. Therefore, the generation of a hydrogen gas or the production of a hydroxide ion upon energization is prevented or at least reduced.

When a layer doped with a minus ion in advance is used as the doping layer 21b of the non-working electrode assembly 20A, energization is caused by the transfer of the minus ion to the skin of an organism and the transfer of a plus ion from the skin of the organism to the doping layer 21b. Even in this case, the generation of a hydrogen gas or the production of a hydroxide ion is prevented or at least reduced.

The non-working electrode assembly 20A has the same constitution as that of the working electrode assembly 10I, so the non-working electrode assembly 20A and the working electrode assembly 10J can be produced by means of the same process. As a result, the production process of an iontophoresis device can be significantly simplified. In addition, the automation of the production of the iontophoresis device and the mass production of the iontophoresis device can be easily performed, and the production cost of the iontophoresis device can be significantly reduced.

The non-working electrode assembly 20B has the electrode 21 identical to that of the non-working electrode assembly 20A and a cation exchange membrane 25C placed on the front surface side of the doping layer 21b.

In the non-working electrode assembly 20B, energization is caused by the transfer of a plus ion from the skin of an organism to the doping layer 21b via the cation exchange membrane 25C such that the layer is doped with the ion. Therefore, the generation of a hydrogen gas or the production of a hydroxide ion upon energization is prevented or at least reduced.

In addition, the non-working electrode assembly 20B is structured in such a manner that the doping layer 21b does not directly contact with a skin. Therefore, a drug can be safely administered even when the doping layer 21b made of a substance which is not preferably brought into contact with an organism is used.

The electrode 21 and the cation exchange membrane 25C can be joined and integrated with each other by means of an approach such as thermocompression bonding. This action can improve energization property between them and the handleability of them.

The non-working electrode assembly 20B has the same constitution as that of the working electrode assembly 10K, so the non-working electrode assembly 20B and the working electrode assembly 10K can be produced by means of the same process. As a result, the production process of an iontophoresis device can be significantly simplified. In addition, the automation of the production of the iontophoresis device and the mass production of the iontophoresis device can be easily performed, and the production cost of the iontophoresis device can be significantly reduced.

The non-working electrode assembly 20C includes: the electrode 21 having the conductive base material 21a connected to the electric supply line 32 and the doping layer 21b formed on the base material 21a and doped with a minus ion; and an anion exchange membrane 25A placed on the front surface side of the doping layer 21b.

In the non-working electrode assembly 20C, when a minus voltage is applied to the electrode 21 in a state where the anion exchange membrane 25A is brought into contact with an organism, a minus ion with which the doping layer 21b is doped transfers to the anion exchange membrane 25A, and the minus ion additionally transfers to the organism, or a counter ion bound to an ion exchange group in the anion exchange membrane 25A and substituted by the minus ion transfers to the organism, whereby energization occurs. Therefore, the generation of a hydrogen gas or the production of a hydroxide ion upon energization is prevented.

The electrode 21 and the anion exchange membrane 25A can be joined and integrated with each other by means of an approach such as thermocompression bonding. This action can improve energization property between them and the handleability of them.

The non-working electrode assembly 20D includes: the electrode 21 identical to that of the non-working electrode assembly 20A; an electrolyte solution holding portion 22 holding an electrolyte solution in contact with the doping layer 21b; and the anion exchange membrane 25A placed on the front surface side of the electrolyte solution holding portion 22.

In the non-working electrode assembly 20D, when a minus voltage is applied to the electrode 21 in a state where the anion exchange membrane 25A is brought into contact with an organism, energization is caused by the transfer of a plus ion in the electrolyte solution holding portion 22 to the doping layer 21b such that the layer is doped with the ion. Therefore, the generation of a hydrogen gas or the production of a hydroxide ion upon energization is suppressed.

Energization between the electrolyte solution holding portion 22 and the skin of the organism is caused by the transfer of a minus ion in the electrolyte solution holding portion 22 to the skin of the organism via the anion exchange membrane 25A.

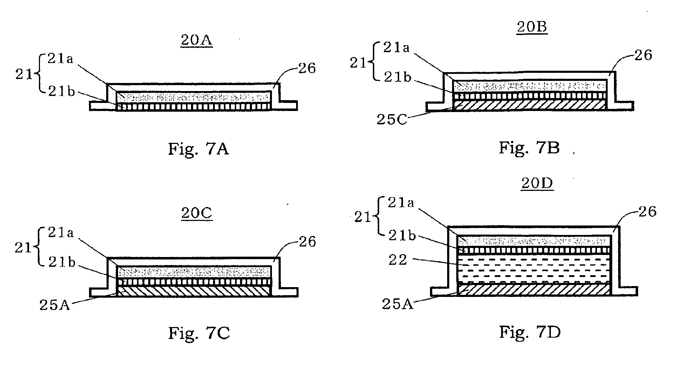

Fig. 7 (A) is a plan view of an electrode 40 to be particularly preferably used as the electrode 11 of each of the working electrode assemblies 10A to 10K or as the electrode 21 of each of the non-working electrode assemblies 20A to 20D, and Fig. 7(B) is a sectional view taken along the line A-A of Fig. 7(A).

In the figures, reference numeral 41 denotes a conductive base material composed of a carbon fiber, and a doping layer 42 made of a conductive polymer or the like is formed on one surface of the base material 41. A terminal member 43 composed of a male fitting portion 43a, a body portion 43b, and a joining portion 43c is attached to the other surface of the base material 41.

The terminal member 43 is obtained by curing, in a die placed on the base material 41, a composition, which is prepared by blending a polymer matrix such as silicon rubber with graphite, black lead, carbon black, or a carbon filler such as fine powder of glass-like carbon or a short fiber obtained by cutting a carbon fiber, through heating and vulcanization. The composition is hardened in a state where it is impregnated into a carbon fiber constituting the base material 41, whereby the base material 41 and the terminal member 43 are integrated with each other at the joining portion 43c.

The electrode 40 enables energization from the doping layer 42 at a uniform current density because a carbon fiber has high conductivity and high flexibility. As a result, the working electrode assemblies 10A to 10K and the non-working electrode assemblies 20A to 20D each having enough flexibility to follow the irregularities of the skin of an organism or the movement of the organism can be realized.

In addition, connection from the electric power source 30 to the electric supply lines 31 and 32 can be performed by means of a connector having a female fitting portion that fits into the male fitting portion 43a. Even when a metallic material is used for the female fitting portion, the metal of the connector is prevented from eluting to transfer to an organism because the male fitting portion 43a is separated from the base material 41 by the body portion 43b.

The terminal member 43 may be attached to the base material 41 by means of an arbitrary method. For example, as shown in Fig. 7(C), the attachment can be performed by: forming engaging portions 43d and 43e on the terminal member 43; and inserting the engaging portion 43e into a small pore arranged on the base material 41.

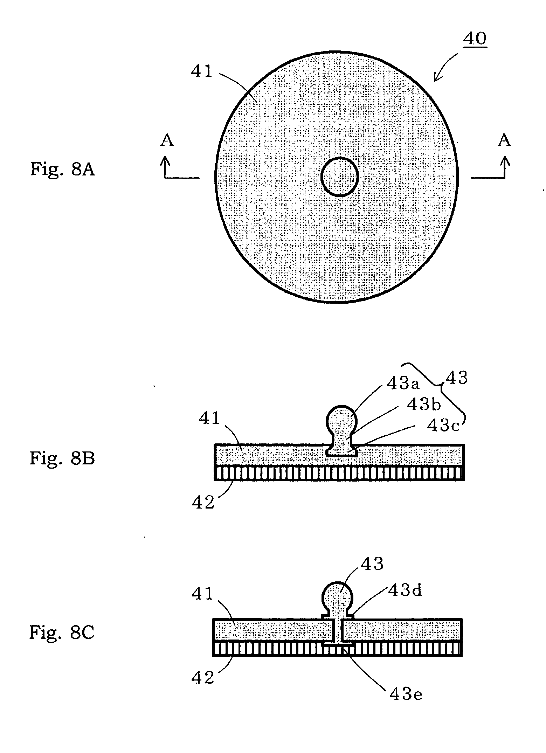

Fig. 8 (A) is a plan view of an electrode 50 according to another aspect to be particularly preferably used as the electrode 11 of each of the working electrode assemblies 10A to 10K or as the electrode 21 of each of the non-working electrode assemblies 20A to 20D, and Fig. 8 (B) is a sectional view taken along the line A-A of Fig. 8 (A) .

In the figures, reference numeral 51 denotes a base material composed of a carbon fiber having a circular conductive sheet portion 51a and an elongated extension portion 51b extending from the conductive sheet portion 51a. A doping layer 52 is formed on one surface of the conductive sheet portion 51a.

The electrode 50 enables energization from the doping layer 52 at a uniform current density as in the case of the electrode 40. As a result, the working electrode assemblies 10A to 10K and the non-working electrode assemblies 20A to 20D each having enough flexibility to follow the irregularities of the skin of an organism or the movement of the organism can be realized.

As shown in Fig. 8(C), the electrode 50 is used in combination with the container 16/26 having an opening 16h/26h formed on the outer peripheral wall 16s/26s or the upper wall 16u/26u, and is housed in the container 16/26 in a state where the extension portion 51b is led from the opening 16h/26h.

Connection from the electric power source 30 to the electric supply lines 31 and 32 can be performed at the led extension portion 51b by means of a connector such as an alligator clip attached to the tip of each of the electric supply lines 31 and 32.

In the case of an iontophoresis device housing therein a member having a high water content such as the electrolyte solution holding portion 12 or 22, or the drug solution holding portion 14 like the working electrode assemblies 10A to 10E and the non-working electrode assembly 20D, a water-repellent portion 51c impregnated with a fluorine-based resin, a silicone-based resin, a silane-based resin, or the like to provide water repellency is arranged at the extension portion 51b placed at the opening 16h or 26h. As a result, water can be prevented from leaking from a working electrode assembly or a non-working electrode assembly. Alternatively, when a metallic member is used for the connector such as an alligator clip, a metal ion eluted from the member can be prevented from penetrating into a working electrode assembly or a non-working electrode assembly.

Each of the base materials 41 and 51 of the electrodes 40 and 50 can achieve the same effect as that described above even when each of the materials is formed of carbon fiber paper. The carbon fiber or carbon fiber paper of the base material 41 or 51 is impregnated with a soft polymer such as silicon rubber or thermoplastic polyurethane, whereby a reduction in quality of an electrode due to the falling of a carbon fiber can be prevented, and the handleability of the electrode 40 or 50 can be improved.

The present invention has been described above by way of several embodiments. However, the present invention is not limited to those embodiments, and can be variously altered within the scope of claims.

For example, the specific shape and dimensions of an electrode assembly, an electrode, or the like are shown merely as examples in each embodiment. The present invention is not limited to the shapes, dimensions, and the like shown in the embodiments.

In addition, in each of the above embodiments, description has been given of the case where a conductive base material having formed thereon a doping layer is used as an electrode. However, the base material is not necessarily conductive, and an electrode can be formed only of a doping layer without the use of a base material.

The iontophoresis device of the present invention can be constituted by combining one or more of the working electrode assemblies 10A to 10K and one or more of the non-working electrode assemblies 20A to 20D. In addition, the iontophoresis device of the present invention can be constituted by combining one or more of the working electrode assemblies 10A to 10K and the non-working electrode assembly 120 or 210 shown in Fig. 9 or 10, or by combining one or more of the non-working electrode assemblies 20A to 20D and the working electrode assembly 110 or 210 shown in Fig. 9 or 10.

Alternatively, a drug can be administered as follows. While any one of the working electrode assemblies 10A to 10K is used, the iontophoresis device itself is provided with no non-working electrode assembly, and, for example, a voltage is applied to the working electrode assembly in a state where the working electrode assembly is brought into contact with the skin of an organism and a part of the organism is brought into contact with a member to serve as the ground. Even in this case, the basic effect of the present invention, that is, the prevention of: the generation of an oxygen gas, a hydrogen gas, a chlorine gas, or the like; or the production of a hydrogen ion, a hydroxide ion, or hypochlorous acid in a working electrode assembly upon energization is achieved. Therefore, such iontophoresis device is also included in the scope of the present invention.

Furthermore, in each of the above embodiments, description has been given of the case where the working electrode assembly, the non-working electrode assembly, and the electric power source are constituted separately. It is also possible that those elements are incorporated in a single casing or an entire device incorporating them is formed in a sheet shape or a patch shape, whereby the handleability thereof is enhanced, and such iontophoresis device is also included in the scope of the present invention.

## Claims

1. An iontophoresis device **characterized by** comprising at least one electrode assembly having an electrode in which a doping layer made of a material effecting an electrochemical reaction owing to doping or de-doping of an ion is formed.

2. The iontophoresis device according to claim 1, **characterized in that** the doping layer contains a conductive polymer.

3. The iontophoresis device according to claim 2, **characterized in that** the conductive polymer comprises polyaniline, polypyrrole, polythiophene or polyacetylene, or a derivative thereof or a mixture thereof.

4. The iontophoresis device according to any one of claims 1 to 3, **characterized in that** the electrode assembly further comprises a drug solution holding portion holding a drug solution containing a drug ion of a first conductivity type, the drug solution holding portion being placed on a front surface side of the doping layer.

5. The iontophoresis device according to claim 4, **characterized in that** the electrode assembly further comprises a first ion exchange membrane of the first conductivity type placed on a front surface side of the drug solution holding portion.

6. The iontophoresis device according to claim 4 or 5, **characterized in that**:
the electrode assembly further comprises a second ion exchange membrane of a second conductivity type placed on the front surface side of the doping layer; and
the drug solution holding portion is placed on a front surface side of the second ion exchange membrane.

7. The iontophoresis device according to any one of claims 1 to 3, **characterized in that** the electrode assembly further comprises:
an electrolyte solution holding portion holding an electrolyte solution, the electrolyte solution holding portion being placed on a front surface side of the doping layer; and
a first ion exchange membrane of the first conductivity type that is placed on a front surface side of the electrolyte solution holding portion and that is doped with a drug ion of the first conductivity type.

8. The iontophoresis device according to claim 7, **characterized in that**:
the electrode assembly further comprises a second ion exchange membrane of the second conductivity type placed on the front surface side of the electrolyte solution holding portion; and
the first ion exchange membrane is placed on a front surface side of the second ion exchange membrane.

9. The iontophoresis device according to claim 7, **characterized in that**:
the electrode assembly further comprises a second ion exchange membrane of the second conductivity type placed on the front surface side of the doping layer; and
the elecrtolyte solution holding portion is placed on a front surface side of the second ion exchange membrane.

10. The iontophoresis device according to any one of claims 1 to 3, **characterized in that**:
the electrode assembly further comprises a first ion exchange membrane of the first conductivity type that is placed on a front surface side of the doping layer and that is doped with a drug ion of the first conductivity type; and
the doping layer is doped with an ion of the first conductivity type.

11. The iontophoresis device according to claim 10, **characterized in that**:
the electrode assembly further comprises a second ion exchange membrane of the second conductivity type placed on a front surface side of the doping layer; and
the first ion exchange membrane is placed on a front surface side of the second ion exchange membrane.

12. The iontophoresis device according to any one of claims 1 to 3, **characterized in that** the doping layer is doped with a drug ion of the first conductivity type.

13. The iontophoresis device according to any one of claims 1 to 3, **characterized in that** the electrode assembly further comprises a first ion exchange membrane of the first conductivity type placed on a front surface side of the doping layer.

14. An iontophoresis device comprising:
a working electrode assembly holding a drug ion of a first conductivity type; and
a non-working electrode assembly as a counter electrode of the working electrode assembly,
**characterized in that** the non-working electrode assembly comprises an electrode in which a doping layer made of a material effecting an electrochemical reaction owing to doping or de-doping of an ion is formed.

15. The iontophoresis device according to claim 14, **characterized in that** the non-working electrode assembly further comprises a third ion exchange membrane of the first conductivity type placed on a front surface side of the doping layer.

16. The iontophoresis device according to claim 14, **characterized in that**:
the no-working electrode assembly further comprises a third ion exchange membrane of the second conductivity type that is placed on the front surface side of the doping layer; and
the doping layer is doped with an ion of the second conductivity type.

17. The iontophoresis device according to any one of claims 1 to 16, **characterized in that**:
the electrode further comprises a conductive base material; and
the doping layer is stacked on the conductive base material.

18. The iontophoresis device according to claim 17, **characterized in that** the conductive base material comprises a conductive sheet made of a carbon fiber or carbon fiber paper.

19. The iontophoresis device according to claim 18, **characterized in that** the electrode further comprises a terminal member with carbon mixed in a polymer matrix, the terminal member being attached to the conductive sheet.

20. The iontophoresis device according to claim 18, **characterized in that** the electrode further comprises an extension portion that is formed integrally with the conductive sheet and that is made of a carbon fiber or carbon fiber paper.
